# EUROPEAN PATENT APPLICATION

(11) **EP 4 643 816 A1**
(43) Date of publication of application: **05.11.2025**
(21) Application number: 24830246.5
(22) Date of filing: 15.05.2024
(51) Int. Cl.: A61C 17/00, G06F 18/213

(54) **METHOD FOR RECOGNIZING ORAL CARE POSITION, AND DEVICE AND STORAGE MEDIUM**

(30) Priority: 29.06.2023 CN 202310794180; 29.06.2023 CN 202310794201
(71) Applicant: Guangzhou Stars Pulse Co., Ltd., Guangzhou, Guangdong 510630 (CN)
(72) Inventor: ZHANG, Zhicheng, Guangzhou, Guangdong 510630 (CN); LIU, Ming, Guangzhou, Guangdong 510630 (CN); WU, Liangyu, Guangzhou, Guangdong 510630 (CN)
(74) Representative: Metida
(86) International application number: PCT/CN2024/093388
(87) International publication number: WO 2025/001598

(57) **Abstract**

An oral care position identification method applied to an oral care device (2000 ) and the oral care device (2000) are provided. The oral care device (2000) includes a handle (130), the care element (110), and a first positioning component. The first positioning component is disposed on the handle (130). The oral care position identification method includes steps of acquiring human body feature information of a user during an oral care process through the first positioning component of the oral care device (2000) and determining a current oral care position corresponding to the care element (110) based on the human body feature information. The care element (110) is within a field of view of the first positioning component, so that the first positioning component is able to acquire feature information of at least part of the care element (110) when the first positioning component is turned on.

## Description

### TECHNICAL FIELD

The present disclosure relates to a technical field of oral care, and in particular to an oral care position identification method, an oral care device, and a computer storage medium.

### BACKGROUND

In an oral care process, most users tend to plan their oral care paths through sub consciousness or muscle memory. Due to different habits of using oral care devices, the users may forget to care for a certain tooth surface, or a cleaning time is not long enough. In the long run, a risk of tooth decay in a tooth area that is missed to be cared increases sharply.

Currently, a mainstream oral care position identification solution is to install a posture sensor on a handle of an oral care device, and then a motion posture algorithm is adopted to identify a posture of the oral care device to determine a current oral care position. However, due to different oral care habits of different users, postures of holding the oral care device vary greatly, which causes some oral care positions to be unable to be identified or causes some oral care positions to be incorrectly identified, and an identification accuracy of the oral care positions is low.

### SUMMARY

Embodiments of the present disclosure provide an oral care position identification method, an oral care device, and a computer storage medium. By configuring a feature acquisition component at a predetermined position of the oral care device or with a predetermined angle relative to the oral care device, the feature acquisition component is able to always acquire feature information of at least part of the care element when the feature acquisition component is turned on. When a user uses the oral care device, the care element needs to be placed in an oral cavity for use. Therefore, during an oral care process, the human body feature information of the user is further acquired through the feature acquisition component, and a current oral care position corresponding to the care element is determined based on the human body feature information, thereby improving effectiveness and accuracy of oral care position identification.

In a first aspect, the embodiments of the present disclosure provide an oral care position identification method applied to an oral care device. The oral care device includes a handle, a care element, and a first positioning component. The first positioning component is disposed on the handle. The first positioning component is configured to acquire images and energy information. When the first positioning component is turned on, the first positioning component is always able to acquire feature information of at least part of the care element. The oral care position identification method includes steps:
acquiring human body feature information of a user during an oral care process through the first positioning component of the oral care device; and
determining a current oral care position corresponding to the care element based on the human body feature information.

In a second aspect, the present disclosure provides an oral care position identification device. The oral care position identification device is applied to an oral care device. The oral care device includes a handle, a care element, and a first positioning component. The first positioning component is disposed on the handle. The first positioning component is configured to acquire images and energy information. When the first positioning component is turned on, the first positioning component is always able to acquire feature information of at least part of the care element. The oral care position identification device includes a first acquiring module and a first determining module. The first acquiring module is configured to acquire human body feature information during an oral care process based on the first positioning component. The first determining module is configured to determine a current oral care position corresponding to the care member based on the human body feature information.

In a third aspect, the present disclosure provides the oral care device. The oral care device includes a memory, a processor, a handle, a care element, and a first positioning component. The first positioning component is disposed on the handle and is configured to acquire images and energy information. When the first positioning component is turned on, the first positioning component is always able to acquire feature information of at least part of the care element. The memory is connected to the processor and is configured to store executable program codes. The processor is configured to run a program corresponding to the executable program codes by reading the executable program codes stored in the memory. The processor is configured to execute steps of acquiring human body feature information of a user during an oral care process through the first positioning component of the oral care device and determining a current oral care position corresponding to the care element based on the human body feature information.

In a fourth aspect, the present disclosure provides the computer storage medium. The computer storage medium includes instructions stored therein. The instructions are loaded by the processor and executed to acquire human body feature information during an oral care process based on a first positioning component of an oral care device, and determine a current oral care position corresponding to a care element of the oral care device based on the human body feature information.

In one or more embodiments of the present disclosure, by enabling the first positioning component being able to always acquire the feature information of at least part of the care element when the first positioning component is turned on, when the user uses the oral care device and places the care element in the oral cavity for use, it is ensured that the first positioning component is able to always track human body features near the oral cavity through the care element. Further, the current oral care position corresponding to the care element is determined according to the human body feature information, thereby avoiding a problem that some oral care positions are unable to be identified due to large differences in different oral care habits of different users in holding postures of the oral care device and avoiding a problem that the human body feature information is unable to be acquired under some holding postures. Therefore, the effectiveness and accuracy of the oral care position identification are improved.

The above description is only an overview of technical solutions of the present disclosure. In order to more clearly understand technical means of the present disclosure, in order to enable the technical solutions to be implemented according to the content of the specification, and in order to achieve the above and other purposes, features, and characteristics of the present disclosure more obvious and easy to understand, the specific implementations of the present disclosure are specifically cited below.

### BRIEF DESCRIPTION OF DRAWINGS

In order to clearly describe technical solutions in the embodiments of the present disclosure, the following will briefly introduce the drawings that need to be used in the description of the embodiments or the prior art. Apparently, the drawings in the following description are merely some of the embodiments of the present disclosure, and those skilled in the art are able to acquire other drawings according to the drawings without contributing any inventive labor.
FIG. 1 is a schematic diagram of an oral care device according to one embodiment of the present disclosure.
FIG. 2 is a schematic diagram of a feature acquisition component acquiring images or light energy information according to one embodiment of the present disclosure.
FIG. 3 is a schematic diagram showing a position of the feature acquisition component according to one embodiment of the present disclosure.
FIG. 4 is a flow chart of an oral care position identification method according to one embodiment of the present disclosure.
FIG. 5 is a schematic diagram showing an alternative position of the feature acquisition component according to one embodiment of the present disclosure.
FIG. 6 is another flow chart of the oral care position identification method according to one embodiment of the present disclosure.
FIG. 7 is a schematic diagram of an implementation process of the oral care position identification method according to one embodiment of the present disclosure.
FIG. 8 is a schematic diagram of another implementation process of the oral care position identification method according to one embodiment of the present disclosure.
FIG. 9 is a schematic diagram of images acquired by the feature acquisition component during an oral care process according to one embodiment of the present disclosure.
FIG. 10 is another flow chart of the oral care position identification method according to one embodiment of the present disclosure.
FIG. 11 is a schematic diagram of the implementation process of the oral care position identification method according to another embodiment of the present disclosure.
FIG. 12 is a flow chart of the oral care position identification method according to another embodiment of the present disclosure.
FIG. 13 is a schematic diagram of the oral care device according to another embodiment of the present disclosure.
FIG. 14 is a flow chart of an oral care area identification method according to another embodiment of the present disclosure.
FIG. 15 is another flow chart of the oral care area identification method according to another embodiment of the present disclosure.
FIG. 16 is another flow chart of the oral care area identification method according to another embodiment of the present disclosure.
FIG. 17 is another flow chart of the oral care area identification method according to another embodiment of the present disclosure.
FIG. 18 is a schematic diagram of an oral care area identification device according to one embodiment of the present disclosure.
FIG. 19 is a schematic diagram of the oral care area identification device according to another embodiment of the present disclosure.
FIG. 20 is a block diagram of the oral care device according to one embodiment of the present disclosure.

### DETAILED DESCRIPTION

In order to make the objectives, technical solutions, and advantages of the embodiments of the present disclosure clear, technical solutions in the embodiments of the present disclosure will be described clearly and completely in conjunction with the accompanying drawings in the embodiments of the present disclosure. Obviously, the described embodiments are only a part of the embodiments of the present disclosure, rather than all of the embodiments. Based on the embodiments of the present disclosure, all other embodiments acquired by those of ordinary skill in the art without creative work shall fall within the protection scope of the present disclosure.

Terms "first", "second", "third", and the like in the specification and claims of the present disclosure and the accompanying drawings are used to distinguish different objects, and are not used to describe a specific order. In addition, terms "include", "have", and any variations thereof are intended to cover non-exclusive inclusion, e.g., includes a series of steps or units, processes, methods, systems, products, or devices, which are not limited to the listed steps or units, but may optionally further include steps or units not listed, or optionally further includes steps or units inherent to the processes, methods, products, or devices.

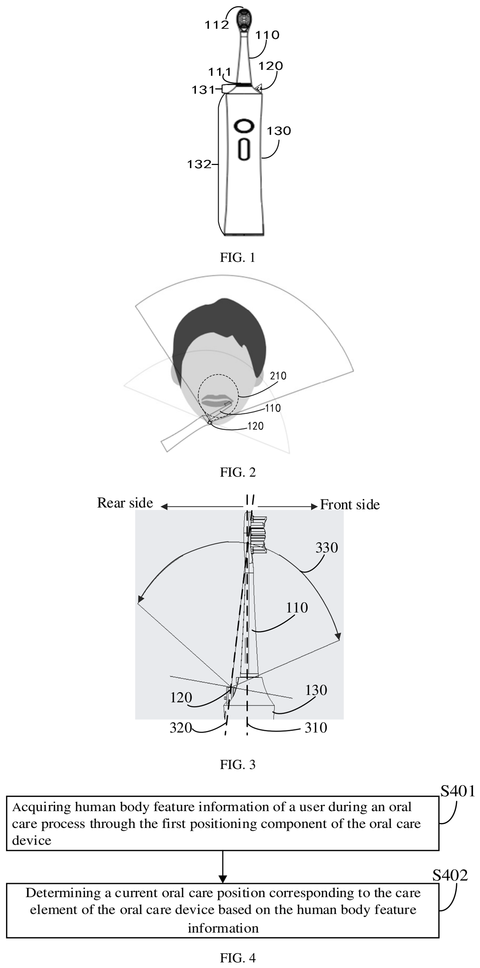

One embodiment of the present disclosure provides an oral care device. The oral care device may be, but is not limited to, an electric toothbrush, an irrigator, a tooth cleaner, an oral detection device, etc. Correspondingly, the care element of the oral care device may be a brush head, a nozzle, a tooth cleaning probe, a detection probe, etc. The care element is configured to extend into an oral cavity of a user when in use. Through the oral care position identification method, the oral care device realizes a cleaning function, a care function, or a detection function while associating a corresponding cleaning action, a corresponding care action, or a corresponding detection action with a corresponding oral care position. FIG. 1 is a schematic diagram of the oral care device according to one embodiment of the present disclosure. As shown in FIG. 1, the oral care device includes a care element 110, a feature acquisition component120, and a handle 130.

The feature acquisition component 120 is disposed on the handle 130. The feature acquisition component 120 may be, but is not limited to, a camera or a photoelectric sensor, etc. The feature acquisition component 120 is configured to acquire mage information or light energy information.

Specifically, when turned on, the feature acquisition component 120 is always able to acquire the feature information of at least part of the care element 110. As shown in FIG. 2, when the user uses the oral care device, the care element 110 needs to be placed in the oral cavity for use. The human body feature information at least includes mouth feature information. The care element 110 may be, but is not limited to, a brush head.

Optionally, the human body feature information includes but is not limited to chin feature information or nose feature information.

Specifically, after the oral care device acquires the human body feature information 210 during an oral care process through the feature acquisition component 120, the oral care device further determines a current oral care position corresponding to the care element 110 based on the human body feature information 210, so as to avoid a problem that some oral care positions are unable to be identified due to large differences in different oral care habits of different users in holding postures of the oral care device, and avoid a problem that the human body feature information is unable to be acquired due to specific oval care postures. Therefore, effectiveness and accuracy of oral care position identification is improved. The human body feature information 210 is acquired from images or light energy information acquired by the feature acquisition component 120. Optionally, the oral care device further includes, but is not limited to, one or more indicator lights, one or more buttons, a speaker, a motor, etc.

In some optional embodiments, as shown in FIG. 1, the care element 110 includes a connecting end 111 and a free end 112 disposed opposite to the connecting end 111. The connecting end 111 of the care element 110 is connected to the handle 130. When the feature acquisition component 120 is turned on, at least an image of a first predetermined range of the care element 120 from the connecting end 111 toward the free end 112 is acquired, so that the feature acquisition component 120 is able to acquire external feature information of the mouth where the care element 110 is used. The first predetermined range is a length starting from the connecting end 111 of the care element 110, such as 0-30 mm from the connecting end 111 of the care element 110 toward the free end of the care element 110. In other words, the first predetermined range is a section of the care element 110 exposed to the mouth when the care element 110 is inserted into the mouth, and a length of the care element inserted into the mouth is commonly within 30 mm. Therefore, the first range starts from a position 30 mm away from the free end 112 to the connecting end 111 of the care element 110. For instance, the first present range is 30-60 mm, 30 -40 mm, 35-50 mm, 40-60 mm, etc. Alternatively, the first predetermined range may include a portion of the care element 110 inserted into the oral cavity. For instance, the first predetermined range is a length of the entire care element 110 or starts from 20 mm away from the free end 112 to the connecting end 111 of the oral element 110, which ensures that when the user uses the oral care device and places the care element 110 in the oral cavity, the feature of the care element 110 is always captured by the feature acquisition component 120.

In other embodiments, when the feature acquisition component 120 is turned on, the feature acquisition component 120 at least acquires an image of a second predetermined range of the care element 110 from the free end 112 to the connection end 111 thereof, so that the feature acquisition component 120 is always able to acquire the care element feature information 110. The second predetermined range may be, but is not limited to, any range of 0-30 mm, such as, but not limited to, 25-30 mm, 10-25 mm, etc. Since a length of an unexposed portion of the care element 110 in the oral cavity is commonly not greater than 30 mm, images acquired by the feature acquisition component 120 always include the care element feature information 110.

In some embodiments, the feature acquisition component 120 is able to capture both the first predetermined range of the care element and the second predetermined range of the care element. For example, the feature acquisition component 120 is able to capture the whole care element 110. It is understood that the first predetermined range and the second predetermined range may be the same or different, which are not limited thereto.

Furthermore, a position of the care element of a predetermined distance from the connecting end to the free end of the care element is located at a center of the image acquired by the feature acquisition component 120. The position of the care element of the predetermined distance from the connecting end to the free end is an exposed position of the care element 110 near the mouth after the care element 110 is placed in the oral cavity. For instance, the position of the care element of the predetermined distance is a position 10 mm, 15mm, 20mm, or 30 mm away from the connecting end 111 of the care element 110. Therefore, it is ensured that effective human body feature information (e.g., the mouth feature information) in each image acquired by the feature acquisition component 120 is close to a central area of each image, which is conducive to the identification of the oral care positions.

In some optional embodiments, as shown in FIG. 1, the handle 130 includes a transition section 131 and a holding section 132. A cross-sectional area of a connecting end of the handle 130 connected to the care element is less than a cross-sectional area of the holding section 132. The transition section 131 connects the holding section 132 to the connecting end of the handle 130. The transition section 131 is a neck or a shoulder of the handle 130. The feature acquisition component 120 is disposed on the transition section 131. Such a configuration enables the feature acquisition component 120 to better acquire the care element feature information 110 and the human body feature information, and effectively avoid obstruction by the handle 130.

In some optional embodiments, as shown in FIG. 3, in a circumferential dimension of the handle 130, when one side where a button is located or a care-oriented surface of the care element 110 is defined as a front side of the handle 130, the feature acquisition component 120 is disposed on a rear side of the handle 130. Therefore, the human body features and the care element 110 during the oral care process are located on the same side of the feature acquisition component 120. The human body features and the care element 110 are located on the same side of the feature acquisition component 120, so it is easy to identify a relative position relationship between the care element 110 and the human body features. Compared with a case where the feature acquisition component 120 is disposed on the front side and the human body features and the care element 110 are disposed on two sides of the feature acquisition component 120, the feature acquisition component 120 is disposed on the rear side of the handle, which not only provides a more accurate reference for a position identification of the care element 110 in the oral cavity, but also enables that the feature acquisition component 120 does not face the user when the user places the oral care device to make the care-oriented surface of the care element facing the teeth. Therefore, privacy of the user is protected.

In some optional embodiments, as shown in FIG. 3, the handle 130 defines an axis 310, the feature acquisition component 120 defines an optical axis 320, the axis 310 intersects the optical axis 320, and at least part of the care element 110 is located within the energy field 330 of the feature acquisition component 120, so as to ensure that the feature acquisition component 120 is always able to acquire the feature information of at least part of the care element 110 when the feature acquisition component 120 is turned on.

Optionally, the oral care device further includes, but is not limited to, a light source configured for supplementing light for the feature acquisition component 120, so that the feature acquisition component 120 is able to acquire clearer images, thereby effectively solving a problem that the oral care position identification is unable to be performed or the accuracy of the oral care position identification is reduced when the oral care device is used in a dark environment.

As shown in FIGS. 1-3, taking the oral care position identification method applied to the oral care device, and a first positioning component being the feature acquisition component as an example, the oral care position identification method of one embodiment of the present disclosure is introduced. FIG. 4 is a flow chart of the oral care position identification method according to one embodiment of the present disclosure. As shown in FIG. 4, the oral care position identification method includes the steps S401-S402.

The step S401 includes acquiring human body feature information of a user during an oral care process through the first positioning component of the oral care device.

Specifically, when the oral care device performs oral care, the first positioning component is turned on, and the images or the light energy information is acquired through the first positioning component during the oral care process. The human body feature information is extracted from the images or the light energy information. The first positioning component may include, but is not limited to, a camera or a photoelectric sensor.

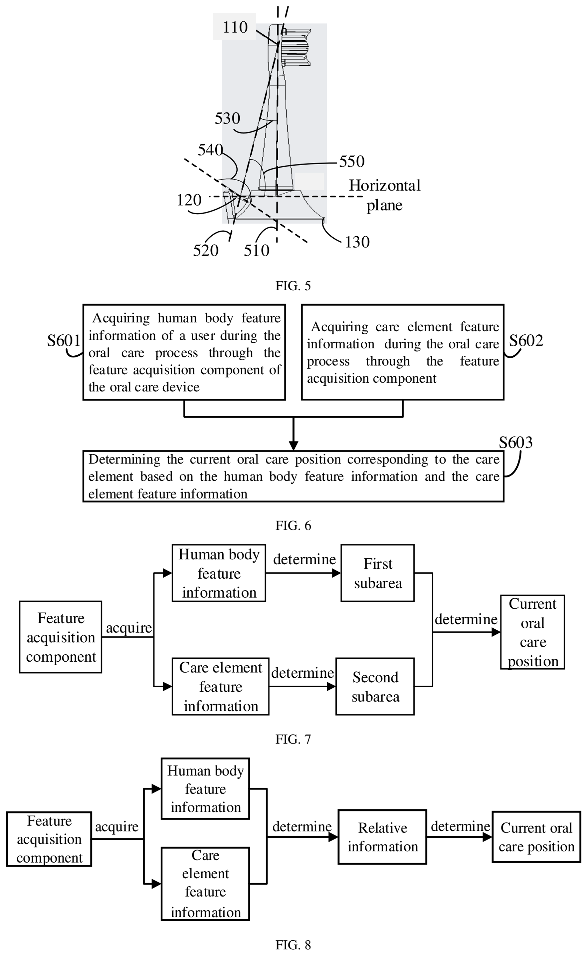

Optionally, as shown in FIG. 5, the care element 110 extends along a first axis 510. The step of acquiring the human body feature information during the oral care process through the first positioning component of the oral care device includes acquiring the human body feature information during the oral care process based on the feature acquisition component 120 disposed on the handle 130 and having an optical axis located at a second axis 520. The included angle 530 between the second axis 520 and the first axis 510 is less than half of a wide angle 540 of the feature acquisition component 120. In this way, it is ensured that the care element 110 is always within the wide angle 540 of the feature acquisition component 120, and the feature acquisition component 120 is always able to acquire at least part of the care element feature information 110.

Furthermore, the included angle between the first axis 510 and the second axis 520 is not greater than 45. That is, the included angle between the optical axis of the feature acquisition component and an extension direction of the care element 110 is within a range of ±45°. On the one hand, it ensures that a common camera is allowed to be installed on the oral care device and serves as a feature acquisition component, thereby ensuring applicability of the oral care position identification method of the embodiment of the present disclosure. On the other hand, a shooting angle of the feature acquisition component is well utilized and the effective human body feature information is shown in a central area of each image.

Optionally, the feature acquisition component 120 may be, but is not limited to, a feature acquisition component, such as the camera, with an energy field of 180°. In one embodiment, the energy field is the wide angle. In order to ensure that the feature acquisition component 120 is able to better acquire the care element feature information 110, as shown in FIG. 5, an inner deviation angle 550 between the optical axis 520 of the feature acquisition component 120 and a horizontal plane is, but is not limited to, in a range of 0-45°.

Optionally, the optical axis of the feature acquisition component 120 is deflected inward relative to the care element 110. That is, the feature acquisition component 120 is disposed toward the care element 110, thereby ensuring that the care element feature information is acquired, and improving the stability of acquiring the images or the light energy information during the oral care process.

The step 402 includes determining a current oral care position corresponding to the care element of the oral care device based on the human body feature information.

Specifically, the human body feature information includes mouth feature information. After the human body feature information is acquired, the current oral care position corresponding to the care element is determined directly based on the mouth feature information of the human body feature information. For instance, the mouth feature information acquired by the feature acquisition component is compared with predetermined mouth feature information respectively corresponding to each of the oral care positions, and a most similar oral care position corresponding to the mouth feature information is determined as the current oral care position corresponding to the care element.

Optionally, the human body feature information further includes, but is not limited to, chin feature information or nose feature information. In order to ensure the accuracy and effectiveness of oral care position identification, the current oral care position corresponding to the care element is further determined based on, but is not limited to, the mouth feature information combined with the chin feature information or the nose feature information acquired by the feature acquisition component.

Optionally, the step of determining the current oral care position corresponding to the care element of the oral care device based on the human body feature information further includes, but is not limited to: inputting the human body feature information into an oral care position identification model, and outputting the current oral care position corresponding to the care element. The oral care position identification model is trained based on a large amount of human body feature information of known oral care positions. In the embodiment, the oral care position identification model is trained by collecting images of the known oral care positions of different users, and the human body feature information in the images of the known oral care positions is associated with corresponding oral care positions. Therefore, when the user performs oral care, the current oral care position corresponding to the care element is determined based on the oral care position identification model by inputting the image/the human body feature information in the image acquired by the feature acquisition component, thereby improving the accuracy and efficiency of the oral care position identification.

Furthermore, since different users have different habits when using the oral care device, the human body feature information acquired at the same oral care position may be different. In order to ensure the accuracy and effectiveness of oral care position identification and avoid the impact of different habits of different uses in using the oral care device on the oral care position identification, the human body feature information includes first human body feature information acquired when performing the oral care by a left hand and second human body feature information acquired when performing the oral care by a right hand. That is, when performing the oral care, the oral care position identification model is able to accurately determine the current oral care position corresponding to the care element by determining whether the human body feature is the first human body feature information or the second human body feature information.

In the embodiment, the feature acquisition component is disposed at a predetermined position or at a predetermined angle, so that the feature acquisition component is always able to acquire the feature information of at least part of the care element when the feature acquisition component is turned on. Therefore, when the user uses the oral care device and places the care element in the mouth for use, it is ensured that the feature acquisition component always tracks the human body feature information of a place where the care element is located during the oral care process through the care element, and determines the current oral care position corresponding to the care element based on the human body feature information. The oral care position identification method avoids a problem that some oral care positions are unable to be identified due to the large differences in postures of holding the oral care device in different oral care habits of different users, thereby improving the effectiveness and accuracy of the oral care position identification.

FIG. 6 is another flow chart of the oral care position identification method according to one embodiment of the present disclosure. As shown in FIG. 6, the oral care position identification method includes steps S601-S603.

The step S601 includes acquiring human body feature information of a user during the oral care process through the feature acquisition component of the oral care device.

Specifically, the step S601 is consistent with the step S401 and is not described in detail herein.

The step S602 includes acquiring care element feature information during the oral care process through the feature acquisition component.

Specifically, when the oral care device performs the oral care, the feature acquisition component is turned on, and images or light energy information during the oral care process is acquired through the feature acquisition component. In addition to extracting the human body feature information from the images or the light energy information, care element feature information during the oral care process is extracted from the images or the light energy information. The feature acquisition component includes, but is not limited to, a camera or a photoelectric sensor.

The step S603 includes determining the current oral care position corresponding to the care element based on the human body feature information and the care element feature information.

Optionally, as shown in FIG. 7, after the human body feature and the care element feature information are acquired through the feature acquisition component, a first subarea is determined based on the human body feature information, and a second subarea is determined based on the care element feature information, and then the current oral care position corresponding to the care element is determined based on the first subarea and the second subarea. For example, but not limited to, the first subarea (i.e., a first tooth area where the care element is currently located in the oral cavity) is determined based on the human body feature information, and the second subarea (i.e., a second tooth area where the care element is currently located in the oral cavity) is determined based on the care element feature information, and then an overlapping area in the first tooth area and the second tooth area or a combined area of the first tooth area and the second tooth area is determined as the current oral care position corresponding to the care element, thereby achieving accurate oral care position identification.

Optionally, as shown in FIG. 8, after the human body features and the care element feature information are acquired through the feature acquisition component, relative positioning information between the care element feature information and the human body feature information is determined first, and then the current oral care position corresponding to the care element is determined based on the relative positioning information between the care element feature information and the human body feature information. That is, in the embodiment of the present disclosure, the oral care position identification is accurately realized based on, but not limited to, a position of the care element (care element feature information) relative to at least one of the human body features of the user (human body feature information, such as the mouth) in the images or the light energy information (relative positioning information) acquired by the feature acquisition component.

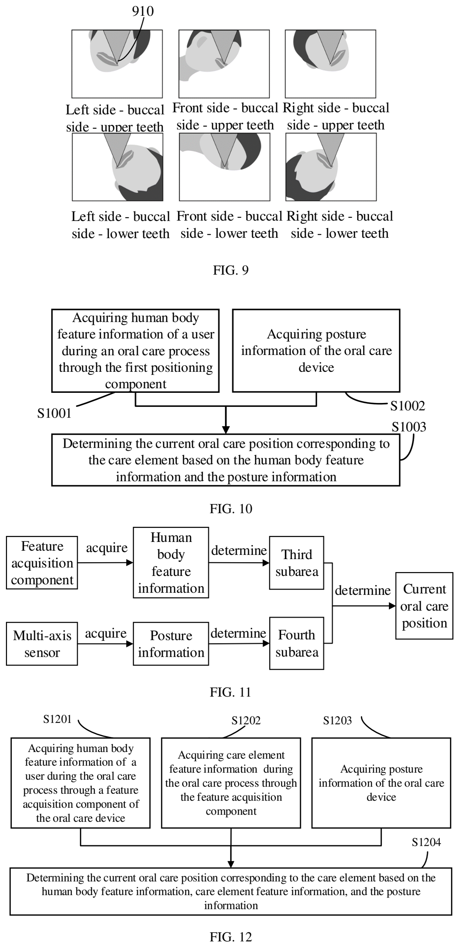

Furthermore, as shown in FIG. 9, a position of the care element 910 relative to an upper lip or a positions of the care element relative to a lower lip corresponding to different tooth areas are also different. Therefore, when determining the current oral care position corresponding to the care element based on the relative positioning information of the care element feature information and the human body feature information, it is possible to determine that the current oral care position is in an upper tooth area or a lower tooth area through the position of the care element in the care element feature information relative to the upper lip in the human body feature information; or determine that the current oral care position is in the upper tooth area or the lower tooth area through the position of the care element in the care element feature information relative to the lower lip in the human body feature information. In the embodiment, the tooth area where the oral care position is located is accurately identified through the position of the care element relative to the upper lip or the lower lip in the images or the light energy information acquired by the feature acquisition component.

For instance, as shown in FIG. 9, when the position of the care element 910 relative to the upper lip is within a predetermined range (that is, a distance between the care element 910 and the upper lip is small) or the position of the care element 910 relative to the lower lip is not within the predetermined range (that is, the distance between the care element 910 and the lower lip is large), it is determined that the current oral care position corresponding to the care element is in the upper tooth area. When the position of the care element 910 relative to the upper lip is not within the predetermined range (i.e., the distance between the care element 910 and the upper lip is large) or the position of the care element 910 relative to the lower lip is within the predetermined range (i.e., the distance between the care element 910 and the lower lip is small), it is determined that the current oral care position corresponding to the care element is in the lower tooth area. When the care element 910 is on the left of the upper lip or the lower lip, it is determined that the current oral care position corresponding to the care element 910 is in a left side of the oral cavity. When the care element 910 is in a middle of the upper lip or the lower lip, it is determined that the current oral care position corresponding to the care element 910 is in the front of the oral cavity. When the care element 910 is located on a right side of the upper lip or the lower lip, it is determined that the current oral care position corresponding to the care element 910 is in a right side of the oral cavity.

It is understood that when determining the current oral care position corresponding to the care element based on the relative positioning information of the care element feature information and the human body feature information, it is also possible to jointly determine whether the current oral care position is in the upper tooth area or the lower tooth area through the position of the care element relative to the upper lip and the lower lip. For example, when it is determined through the position of the care element relative to the upper lip and the lower lip that the care element is closer to the upper lip, it is determined that the current oral care position corresponding to the care element is in the upper tooth area. Alternatively, when it is determined through the position of the care element relative to the upper lip and the lower lip that the care element is closer to the lower lip, it is determined that the current oral care position corresponding to the care element is in the lower tooth area, etc., which is not limited thereto.

In the embodiment, the human body feature information and the care element feature information are acquired during the oral care process through the feature acquisition component, and the current oral care position corresponding to the care element is determined based on the human body feature information and the care element feature information, thereby enabling accurate oral care position identification to be achieved through the human body feature information and the care element feature information, further improving the effectiveness and accuracy of oral care position identification.

As shown in FIG. 10, the oral care position identification method includes steps S1001-S1003.

The step S1001 includes acquiring human body feature information of a user during an oral care process through the first positioning component.

Specifically, the step S1001 is consistent with the step S401 and is not described in detail herein.

The step S1002 includes acquiring posture information of the oral care device.

Specifically, in addition to the feature acquisition component (i.e., the first positioning component), the oral care device further includes a multi-axis sensor. When the oral care device performs oral care work, the posture information of the oral care device is acquired through the multi-axis sensor. The multi-axis sensor includes, but is not limited to, a three-axis acceleration sensor and/or a three-axis gyroscope sensor. The posture information includes, but is not limited to, the acceleration and direction of the oral care device in three-dimensional space and/or the rotation angle, rotation speed and rotation axis of the oral care device in three-dimensional space.

The step S1003 includes determining the current oral care position corresponding to the care element based on the human body feature information and the posture information.

Optionally, as shown in FIG. 11, during the oral care process, after acquiring the human body feature information through the feature acquisition component and after acquiring the posture information of the oral care device through the multi-axis sensor, a third subarea is determined based on the human body feature information, and a fourth subarea is determined based on the posture information. Then the current oral care position corresponding to the care element is determined based on the third subarea and the fourth subarea. Since a human face is symmetrical, left side information and right side information of the human face in the human body feature information acquired are symmetrical. Therefore, whether the care element is currently in the upper tooth area or the lower tooth area (the third subarea) is determined through the human body feature information, The orientation of the care element is determined through the posture information of the oral care device. Therefore, whether the care element is currently in the left tooth area, the right tooth area, or a middle tooth area (the fourth subarea) is accurately determined. Finally, the third subarea and the fourth subarea are combined to acquire the current oral care position corresponding to the care element.

Optionally, during the oral care process, after acquiring the human body feature information and the posture information of the oral care device, a first current oral care position corresponding to the care element is determined based on the human body feature information, and a second current oral care position corresponding to the care element is determined based on the posture information of the oral care device. Then, the current oral care position corresponding to the care element is determined based on the first current oral care position and the second current oral care position. For example, the current oral care position corresponding to the care element is an overlapping position of the first current oral care position and the second current oral care position, which is not limited thereto.

In the embodiment, during the oral care process, the human body feature information is acquired by the feature acquisition component, and the posture information of the oral care device is acquired by the multi-axis sensor, so as to achieve accurate identification of the current oral care position corresponding to the care element based on a dual calibration of the human body feature information and the posture information.

FIG. 12 is a flow chart of the oral care position identification method according to another embodiment of the present disclosure. As shown in FIG. 12, the oral care position identification method includes steps S 1201-S 1204.

The step S1201 includes acquiring human body feature information of a user during the oral care process through a feature acquisition component of the oral care device.

Specifically, the step S1201 is consistent with the step S401 and is not described in detail herein.

The step S1202 includes acquiring care element feature information during the oral care process through the feature acquisition component.

Specifically, the step S1202 is consistent with the step S602 and is not described in detail herein.

The step S1203 includes acquiring posture information of the oral care device.

Specifically, the step S1203 is consistent with the step S1002 and is not described in detail herein.

The step S 1204 includes determining the current oral care position corresponding to the care element based on the human body feature information, care element feature information, and the posture information.

Optionally, after acquiring the human body feature information and the care element feature information during the oral care process through the feature acquisition component, and after acquiring the posture information of the oral care device during the oral care process through the multi-axis sensor, a fifth subarea is determined based on the human body feature information, a sixth subarea is determined based on the care element feature information, and a seventh subarea is determined based on the posture information. Then, the current oral care position corresponding to the care element is determined based on the fifth subarea, the sixth subarea and the seventh subarea. The current oral care position corresponding to the care element may be, but is not limited to, an overlapping position between the fifth subarea, the sixth subarea and the seventh subarea in the oral cavity, so that the tooth area where the care element is located that is determined by the human body feature information, the care element feature information, and the posture information is accurately identified.

Optionally, after acquiring the human body feature information and the care element feature information during the oral care process through the feature acquisition component, and after acquiring the posture information of the oral care device during the oral care process through the multi-axis sensor, an eighth subarea is determined based on the human body feature information and the care element feature information, and a ninth subarea is determined based on the posture information. Then, the current oral care position corresponding to the care element is determined based on the eighth subarea and the ninth subarea. The current oral care position corresponding to the above-mentioned care element may be, but is not limited to, an overlapping position between the eighth subarea and the ninth subarea in the oral cavity, so that whether the care element is currently in the upper tooth area or the lower tooth area is accurately identified through the human body feature information and the care element feature information, and whether the care element is currently in the left tooth area, the middle tooth area, or the right tooth area is accurately identified through the posture information, thereby improving the effectiveness and accuracy of the oral care position identification.

In the embodiment, during the oral care process, the human body feature information is acquired through the feature acquisition component and the posture information of the oral care device is acquired through the multi-axis sensor, thereby further improving the accuracy and effectiveness of the oral care position identification based on the dual calibration of the human body feature information and the posture information.

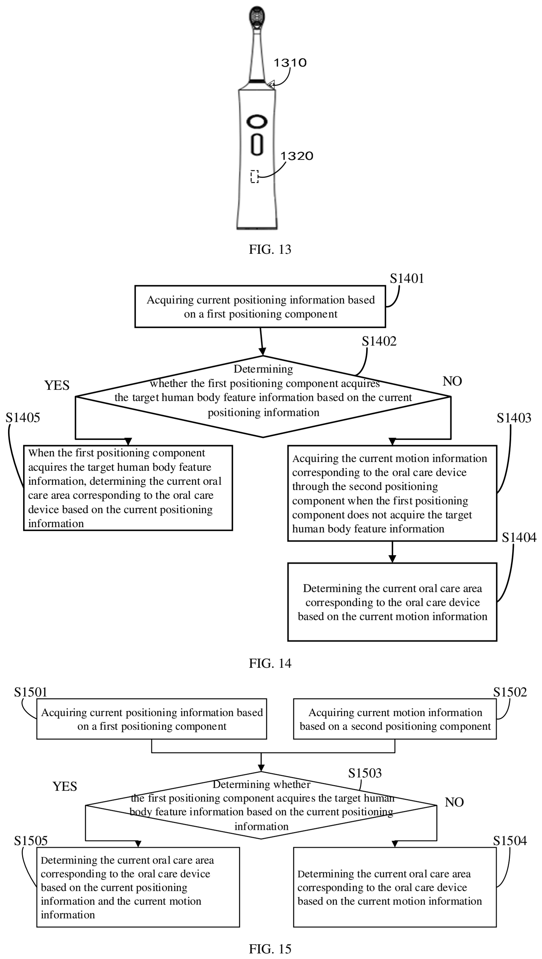

Next, an oral care device provided by one embodiment of the present disclosure is introduced. FIG. 13 is a schematic diagram of the oral care device according to another embodiment of the present disclosure. As shown in FIG. 13, the oral care device includes: a first positioning component 1310 and a second positioning component 1320. The first positioning component 1310 is the feature acquisition component mentioned above.

The first positioning component 1310 may be, but not limited to, a camera, a structured light sensor, etc. The first positioning component 1310 is configured to acquire current positioning information corresponding to the oral care device during an oral care process. The current positioning information may be, but is not limited to, a current oral care image or current light energy information.

The second positioning component 1320 is a compensation sensor, such as, but not limited to, a multi-axis sensor, a pressure sensor, a micro radar, etc. The second positioning component 1320 is configured to acquire current motion information corresponding to the oral care device. The current motion information may be, but is not limited to, at least one of posture information and displacement information. The displacement information includes a moving direction, a flip angle, and a displacement distance.

Specifically, when the oral care device performs oral care, the current positioning information is acquired based on the first positioning component 1310, and it is determined whether the first positioning component 1310 acquires the target human feature information based on the current positioning information. When the first positioning component 1310 does not acquire the target human feature information, it is considered that the first positioning component 1310 is blocked, and the current motion information corresponding to the oral care device is acquired based on the second positioning component 1320. The current oral care area corresponding to the oral care device is determined based on the current motion information, so that when the first positioning component 1310 is unable to acquire the target human feature information, the current oral care area is effectively recognized, thereby avoiding a problem that the first positioning component 1310 of the oral care device cannot acquire the target human feature information. That is, the current positioning information acquired by the first positioning component 1310 is invalid or inaccurate, resulting in the failure or inaccuracy of the oral care area identification, and ensuring the stability and accuracy of the oral care area identification. The target human body feature information is extracted from the current positioning information acquired by the first positioning component 1310.

Optionally, the oral care device further includes one or more indicator lights, one or more buttons, speakers, motors, etc, which is not limited thereto.

Next, in conjunction with FIG. 13, an oral care area identification method provided by another embodiment of the present disclosure is introduced by taking the oral care area identification method applied to the oral care device as an example. FIG. 14 is a flow chart of an oral care area identification method according to another embodiment of the present disclosure. As shown in FIG. 15, the oral care area identification method includes steps S1401-S1405.

The step S 1401 includes acquiring current positioning information based on a first positioning component.

Specifically, when the user uses the oral care device for oral care, the oral care device acquires the current positioning information corresponding to the oral care device through the first positioning component, and the current positioning information includes but is not limited to the current oral care image or current light energy information corresponding to the oral care device. The first positioning component includes at least one of an image acquisition component (such as the camera) and a photoelectric sensor (such as a structured light sensor).

In one embodiment, when the first positioning component is not blocked, it is considered that the first positioning component currently acquires valid current positioning information. Namely, the current positioning information acquired by the first positioning component includes target human body feature information for oral care area identification, and the first positioning component is controlled to acquire the current positioning information with a first power consumption. When the first positioning component is blocked, it is considered that the current positioning information acquired by the first positioning component is invalid. Namely, the current positioning information acquired by the first positioning component does not include target human body feature information and is not configured for the oral care area identification. At this time, the first positioning component is controlled to acquire the current positioning information with a second power consumption less than the first power consumption. That is, when the first positioning component is not blocked, the first positioning component is controlled to acquire the current positioning information with normal power consumption (the first power consumption), and after the first positioning component is blocked, the first positioning component is controlled to work with low power consumption (the second power consumption), and when the first positioning component is restored and is blocked to acquire valid current positioning information with the normal power consumption (the first power consumption). On the one hand, such arrangement reduces waste of power consumption and enhances battery life of the oral care device when the first positioning component is blocked and causes invalid current positioning information. On the other hand, the first positioning component is able to normally acquire the valid current positioning information when the first positioning component is not blocked, thereby ensuring normal and effective identification of the current oral care area.

It should be noted that, in the embodiment, whether the first positioning component is blocked or not does not depend on an exposed area of the first positioning component, but depends on whether the first positioning component is able to effectively acquire images or light energy information.

The step S1402 includes determining whether the first positioning component acquires the target human body feature information based on the current positioning information.

Specifically, after the first positioning component acquires the current positioning information, in order to determine whether the current positioning information is effectively used for the identification of the current oral care area, it is first determined whether the current positioning information includes the target human body feature information. The target human body feature information is the valid human body feature information, such as, but not limited to the human body feature information of the head, the human face, eyes, the mouth, the nose of the user during the oral care process.

The step S1403 includes acquiring the current motion information corresponding to the oral care device through the second positioning component when the first positioning component does not acquire the target human body feature information.

Specifically, when the first positioning component does not acquire the target human body feature information, that is, there is no effective human body feature information in the current positioning information, it is determined that the first positioning component is blocked, and the current oral care area is unable to be effectively and accurately identified through the current positioning information acquired by the first positioning component. At this time, the second positioning component is controlled to acquire the current motion information corresponding to the oral care device. The second positioning component includes at least one of a multi-axis sensor, a motion sensor, a pressure sensor, and a micro radar, which is not limited thereto.

Optionally, when the first positioning component is blocked, the first positioning component is unable to acquire the effective target human body feature information, that is, the current positioning information acquired by the first positioning component is invalid. When the first positioning component is blocked, a current oral care image or current light energy information corresponding to the blocked part remains unchanged or changes slightly. Namely, when a proportion of predetermined information in the current positioning information acquired by the first positioning component is greater than a predetermined proportion threshold and a change amplitude within a first predetermined time is less than a first predetermined value, it is determined that the first positioning component is blocked. When the current positioning information is the current oral care image, the predetermined information may be an image composed of pure color blocks in the current oral care image, such as but not limited to pure black blocks, pure white blocks, pure honeycomb color blocks, pure flesh-colored color blocks, etc. The proportion of the predetermined information in the current positioning information is a proportion of the pure color blocks in the current oral care image to color blocks in the current oral care image. When the current positioning information is the current light energy information, the predetermined information may be the light energy information in the current light energy information that is not greater than the predetermined energy. The predetermined proportion threshold may be, but not limited to, 50%, 60%, etc. For example, when the first positioning component is blocked by foam and the first positioning component is the camera, the features in the oral care images that correspond to a portion of the first positioning component blocked by the foam remain unchanged for a certain period of time.

Optionally, in order to avoid misjudgment of whether the first positioning component is blocked, the first predetermined time should be greater than the time when the care element is placed in a certain position when brushing teeth. For example, the user usually brushes his/her teeth by slowly moving the oral care device, or by briefly remaining the care element in the certain position to let a brush head vibrate. The stay time of the care element in the certain position is commonly not greater than 3 seconds (s). Therefore, the first predetermined time is set to be greater than 3s, such as 4s, 5s, 6s, etc. thereby avoiding a problem that many coral care images having little changer are taken within the first predetermined time to cause misjudgment of the first positioning component being blocked.

Optionally, the motion of the electric toothbrush identified by the second positioning component is also able to determine whether the first positioning component is blocked. For example, when the second positioning component identifies that the electric toothbrush is in motion, but the images or the light energy information acquired by the first positioning component does not change or changes insignificantly, it is determined that the first positioning component is blocked. When the second positioning component identifies that the electric toothbrush is in a stationary state, the images or the light energy information acquired by the first positioning component does not change, and the first positioning component is not determined to be blocked.

After the step S1402 of determining whether the first positioning component acquires the target human body feature information based on the current positioning information, when the first positioning component does not acquire the target human body feature information, that is, there is no effective human body feature information in the current positioning information, it is determined that the first positioning component is blocked, and a prompt message is issued to prompt the user that the first positioning component of the oral care device is blocked and the current oral care area is unable to be identified through the first positioning component.

The step S1404 includes determining the current oral care area corresponding to the oral care device based on the current motion information.

Specifically, the current motion information includes but is not limited to at least one of the posture information and the displacement information. The posture information includes an inclined angle and a bristle orientation of the care element, and the displacement information includes the moving direction, the flip angle and the displacement distance.

In the embodiment, the current positioning information is first acquired through the first positioning component of the oral care device, and it is determined whether the first positioning component has acquired the target human body feature information based on the current positioning information. When the first positioning component does not acquire the target human body feature information, the current motion information corresponding to the oral care device is acquired based on the second positioning component, and the current oral care area corresponding to the oral care device is determined based on the current motion information, so that when the first positioning component is unable to acquire the target human body feature information, the current oral care area can still be effectively identified, thereby avoiding the problem that the first positioning component of the oral care device is unable to acquire the target human body feature information (i.e., the current positioning information acquired by the first positioning component is invalid or inaccurate, resulting in the failure or inaccuracy of the oral care area identification) and ensuring the stability and accuracy of the oral care area identification.

Of course, in other embodiments, when the first positioning component is not blocked, the first positioning component and the second positioning component jointly acquire information to determine the current oral care area.

Optionally, after the current motion information corresponding to the oral care device is acquired through the second positioning component, the current oral care area corresponding to the oral care device is determined based on an initial oral care position acquired by the first positioning component and the current motion information. Namely, an oral care position of the oral care device identified by the first positioning component is first served as the initial oral care position, and then the current oral care area corresponding to the oral care device is identified by the current motion information acquired by the second positioning component, such as the moving direction and displacement distance of the oral care device. Therefore, when the first positioning component is blocked and the target human body feature information is not acquired, the oral care area is still effectively identified, avoiding the problem of invalid or inaccurate oral care area identification caused by invalid or inaccurate current positioning information acquired by the first positioning component, and the stability and accuracy of oral care area identification is further improved by combining the initial oral care position and current motion information of the oral care device.

As shown in FIG. 14, after the step S1402 of determining whether the first positioning component acquires the target human body feature information based on the current positioning information, the oral care area identification method further includes a step S1405.

The step S1405 includes when the first positioning component acquires the target human body feature information, determining the current oral care area corresponding to the oral care device based on the current positioning information.

Specifically, when the first positioning component acquires the target human body feature information, that is, the current positioning information includes the effective human body feature information, it is considered that the first positioning component is not blocked, and the current oral care area corresponding to the oral care device is directly determined based on the current positioning information. Therefore, when the first positioning component is not blocked, the effective and accurate oral care area identification is directly realized through the effective current positioning information without using the second positioning component. The determination of the current oral care area corresponding to the oral care device based on the current positioning information includes, but is not limited to, inputting the current positioning information into the oral care position identification model and outputting a corresponding current oral care area. The oral care position identification model is trained based on the positioning information of a plurality of known oral care areas.

Specifically, when the first positioning component is the camera, the current positioning information is the current oral care image. As shown in FIG. 9, when the user is caring for different oral care areas, the oral care images corresponding to the first positioning component are obviously different. For example, for different tooth areas, face ranges in the oral care images corresponding to the first positioning component are different, or the flip angles of faces in the oral care images are different, or relative positions between the brush head 910 and the upper lip or the relative positions between the brush head and the lower lip are different. When the first positioning component is the camera and is not blocked, the current oral care image (current positioning information) is acquired through the camera, and the current oral care image includes but is not limited to the target human body feature information and the brush head feature information. When determining the current oral care area corresponding to the oral care device based on the current positioning information, the corresponding current oral care area is determined, but is not limited to, by determining the corresponding current oral care area according to the face range in the current oral care image or the flip angle, or by determining whether the current oral care area is in the upper teeth area or the lower teeth area through the relative position of the brush head feature information in the current positioning information and the upper lip in the target body feature information; or, determining whether the current oral care area is in the upper teeth area or the lower teeth area through the relative position of the brush head feature information in the current positioning information and the lower lip in the target body feature information, etc.

FIG. 15 is another flow chart of the oral care area identification method according to another embodiment of the present disclosure. As shown in FIG. 15, the oral care area identification method includes steps S1501-S1505.

The step S1501 includes acquiring current positioning information based on a first positioning component.

Specifically, the step S1501 is consistent with the step S1401 and is not described in detail herein.

The step S1502 includes acquiring current motion information based on a second positioning component.

Specifically, after the power of the oral care device is turned on, the second positioning component is directly turned on to acquire the current motion information corresponding to the oral care device. Namely, no matter whether the first positioning component is blocked or not, the second positioning component always works.

The step S1503 includes determining whether the first positioning component acquires the target human body feature information based on the current positioning information.

Specifically, the step S1503 is consistent with the step S1402 and is not described in detail herein.

It is understood that the step S1503 and the step S1502 may be executed simultaneously or successively. Namely, after executing the step S1503 and determining whether the first positioning component acquires the target human body feature information based on the current positioning information, the step S1502 is executed to acquire the current motion information corresponding to the oral care device through the second positioning component, and then whether to execute the step S1505 or the step S1504 is determined according to a determined result in the step S1503, which is not limited thereto.

The step S1504 includes determining the current oral care area corresponding to the oral care device based on the current motion information.

Specifically, the step S1504 is consistent with the step S1404 and is not described in detail herein.

As shown in FIG. 15, after the step S1503 of determining whether the first positioning component acquires the target human body feature information based on the current positioning information, the oral care area identification method further includes a step S1505.

The step S1505 includes when the first positioning component acquires the target human body feature information, determining the current oral care area corresponding to the oral care device based on the current positioning information and the current motion information.

Specifically, when the first positioning component acquires the target human body feature information, it is determined that the first positioning component is not blocked and the current positioning information acquired by the first positioning component is effective. Then, the current oral care area corresponding to the oral care device is determined based on the current positioning information and the current motion information. For example, but not limited to, a first oral care area corresponding to the oral care device is determined based on the current positioning information, and a second oral care area is determined corresponding to the oral care device based on the current motion information, and then the current oral care area corresponding to the oral care device is determined based on both the first oral care area and the second oral care area. Thus, cross-validation identification is performed through the current positioning information and the current motion information, which further improves the accuracy of the oral care area identification. The current oral care area is, but is not limited to, an overlapping area or a combined area of the first oral care area and the second oral care area.

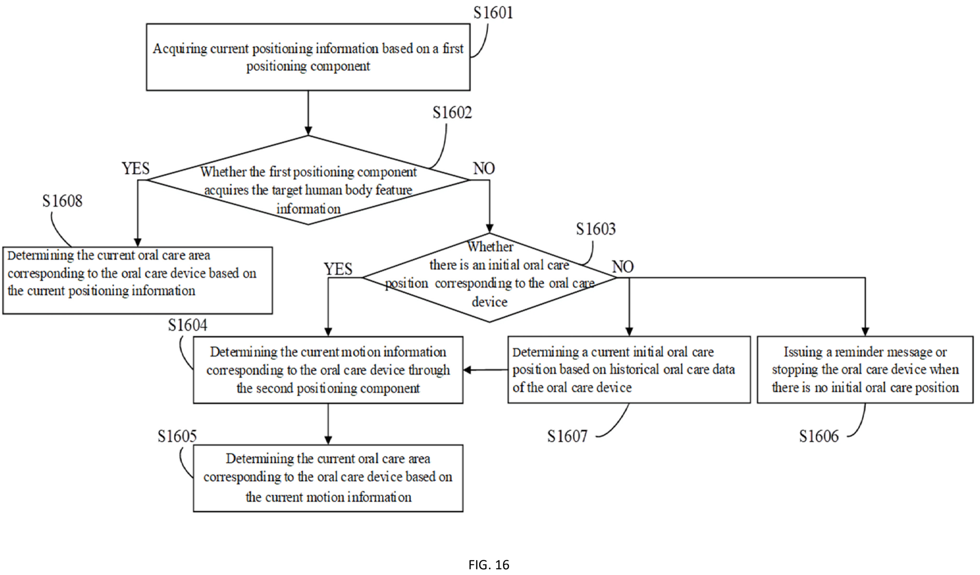

FIG. 16 is another flow chart of the oral care area identification method according to another embodiment of the present disclosure. As shown in FIG. 16, the oral care area identification method includes steps S 1601-S1605.

The step S1601 includes acquiring current positioning information based on a first positioning component.

Specifically, the step S1601 is consistent with the step S1401 and is not described in detail herein.

The step S1602 includes determining whether the first positioning component acquires the target human body feature information based on the current positioning information.

Specifically, the step S1602 is consistent with the step S1402 and is not described in detail herein.

The step S1603 includes when the first positioning component does not acquire the target human body feature information, determining whether there is an initial oral care position corresponding to the oral care device.

Specifically, when the first positioning component does not acquire the target human body feature information, that is, there is no effective human body feature information in the current positioning information, it is considered that the first positioning component is blocked, and it is determined whether there is the initial oral care position corresponding to the oral care device before the first positioning component is blocked.

Optionally, when the first positioning component does not acquire the target human body feature information, in addition to determining whether there is the initial oral care position corresponding to the oral care device, the initial oral care position corresponding to the oral care device is also allowed to be directly determined based on historical positioning information acquired by the first positioning component, and the historical positioning information is positioning information acquired before the first positioning component acquires the target human body feature information. That is, when the first positioning component is blocked and does not acquire the target human body feature information, a historical oral care position acquired by the first positioning component before the first positioning component is blocked is directly served as the initial oral care position, thereby providing a basis for accurately and effectively realizing the identification of the current oral care area through the second positioning component when the first positioning component is blocked.

The step S1604 includes when there is the initial oral care position, determining the current motion information corresponding to the oral care device through the second positioning component.

Specifically, when there is the initial oral care position before the first positioning component acquires the target human body feature information, the current motion information corresponding to the oral care device is acquired through the second positioning component, so that the second positioning component acquires accurate and effective current motion information corresponding to the oral care device in combination with the initial oral care position of the oral care device, thereby improving the accuracy and effectiveness of oral care area identification.

The step S1605 includes determining the current oral care area corresponding to the oral care device based on the current motion information.

Specifically, the step S1605 is consistent with the step S1404 and is not described in detail herein.

As shown in FIG. 16, after the step 1603 of determining whether there is an initial oral care position corresponding to the oral care device when the first positioning component does not acquire the target human body feature information, the oral care area identification method further includes a step S1606.

The step S1606 includes issuing a reminder message or stopping the oral care device when there is no initial oral care position.

Specifically, if the first positioning component does not acquire the target human body feature information and the oral care device does not have the initial oral care position, the reminder message is issued or the oral care device stops working, thereby reminding the user that the current oral care area identification is not effectively identified or inaccurate. Therefore, the user ca realize that am oral care area identification function of the oral care device needs to be adjusted and checked.

As shown in FIG. 16, after the step S1603 of when the first positioning component does not acquire the target human body feature information, determining whether there is an initial oral care position corresponding to the oral care device. In addition to executing the step S1606, the oral care area identification method further includes a step S1607.

The step S1607 includes when there is no initial oral care position, determining a current initial oral care position based on historical oral care data of the oral care device.

Specifically, when the first positioning component does not acquire the target human body feature information, and the oral care device does not have the initial oral care position, it is determined that the first positioning component has been blocked before the user starts an oral care operation, and the current initial oral care position is directly determined based on the historical oral care data of the oral care device. The current initial oral care position may be, but is not limited to, an oral care position with the highest frequency of being marked as the initial oral care position in the historical oral care data when the oral care device starts the oral care, or an oral care position corresponding to a last or previous oral care work of the oral care device in the historical oral care data, thereby ensuring that even if the first positioning component is blocked before the oral care starts, the initial oral care position of the oral care device is determined. In this way, the accuracy of the oral care area identification is ensured based on the current motion information acquired by the second positioning component, and the stability and effectiveness of the oral care area identification is ensured.

Optionally, during the oral care process of the oral care device, when the first positioning component is blocked by the foam or the hand of the user, in order to ensure the stability and effectiveness of the oral care area identification, an oral care guiding position displayed in a display area of a terminal connected to the oral care device or a display area of the oral care device is directly served as the current initial oral care position corresponding to the oral care device.

The step A1608 includes when the first positioning component acquires the target human body feature information, determining the current oral care area corresponding to the oral care device based on the current positioning information.

Specifically, the step S1608 is consistent with the step S1405 and is not described in detail herein.

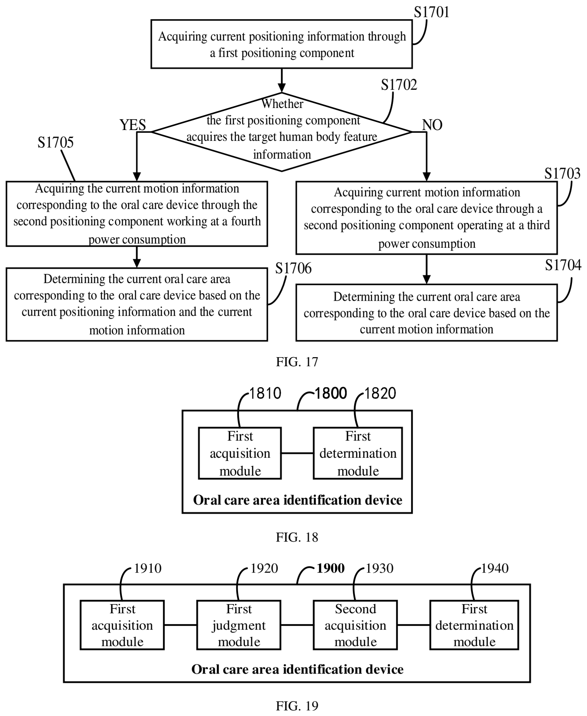

FIG. 17 is another flow chart of the oral care area identification method according to another embodiment of the present disclosure. As shown in FIG. 17, the oral care area identification method includes steps S1701-S1706.

The step S1701 includes acquiring current positioning information based on a first positioning component.

Specifically, the step S1701 is consistent with the step S1401 and is not described in detail herein.

The step S1702 includes determining whether the first positioning component acquires the target human body feature information based on the current positioning information.

Specifically, the step S1702 is consistent with the step S1402 and is not described in detail herein.

The step S1703 includes when the first positioning component fails to acquire the target human body feature information, acquiring current motion information corresponding to the oral care device through a second positioning component operating at a third power consumption.

The step S1704 includes determining the current oral care area corresponding to the oral care device based on the current motion information.

Specifically, the step S1704 is consistent with the step S1404 and is not described in detail herein.

As shown in FIG. 16, after the step S1702 of determining whether the first positioning component acquires the target human body feature information based on the current positioning information, the oral care area identification method further includes the steps S1705 and S1706.

The step S1705 includes when the first positioning component acquires the target human body feature information, acquiring the current motion information corresponding to the oral care device through the second positioning component working at a fourth power consumption.

Specifically, when the first positioning component does not acquire the target human body feature information, it is determined that the first positioning component is blocked, and the current motion information corresponding to the oral care device is acquired through the second positioning component working at the third power consumption. When the first positioning component acquires the target human body feature information, that is, the first positioning component is not blocked, the current motion information corresponding to the oral care device is acquired based on the second positioning component working at the fourth power consumption. The fourth power consumption is less than the third power consumption. In order to avoid unnecessary power consumption of the second positioning component of the oral care device when the first positioning component is not blocked, thereby enhancing the endurance of the oral care device.

The step S1706 includes when the first positioning component acquires the target human body feature information, determining the current oral care area corresponding to the oral care device based on the current positioning information and the current motion information.

Specifically, the step S1706 is consistent with the step S1505 and is not described in detail herein.

In some optional embodiments, the step of acquiring the current positioning information through the first positioning component includes: after the oral care device starts the oral care work, controlling the oral care device to acquire the current positioning information through the first positioning component and start the oral care area identification. Specifically, after a motor of the oral care device starts to vibrate, after a pressure sensor of the oral care device detects a pressure, or after a contact sensor of the oral care device detects that the oral care device is touched, it is considered that the oral care device starts the oral care work and the user uses the oral care device to start the oral care operation, so as to avoid invalid oral care area identification before the oral care device is turned on or before the oral care work is started. Thus, the endurance of the oral care device is not affected. When the first positioning component does not acquire the target human body feature information and the oral care device stops vibrating, the oral care device is controlled to stop the oral care area identification. For example, the second positioning component is controlled to stop acquiring the current motion information corresponding to the oral care device, the first positioning component is controlled to stop acquiring the current positioning information, etc., so as to avoid the oral care device performing the oral care area identification o when the oral care operation is not performed. Therefore, waste of power consumption is avoided, and the endurance of the oral care device is enhanced.

In some optional embodiments, the current motion information includes a displacement distance. After the current motion information corresponding to the oral care device is acquired through the second positioning component, when the displacement distance is greater than a second predetermined value or the oral care device stops vibrating, it is determined that the oral care device has been removed from the oral cavity and no oral care operation is required. The second positioning component is controlled to stop acquiring the current motion information corresponding to the oral care device, so that after the oral care device stops vibrating or the user does not perform the oral care operations, the oral care device stops identifying the oral care areas, further avoiding invalid identification of the oral care areas and ensuring the endurance of the oral care device.

FIG. 18 is a schematic diagram of an oral care position identification device according to one embodiment of the present disclosure. The oral care position identification device is applied to an oral care device. The oral care device includes a handle, a care element, and a first positioning component. The first positioning component is a feature acquisition component. The feature acquisition component is disposed on the handle and is configured to acquire images or light energy information, and the feature acquisition component is able to always acquire feature information of at least part of the care element when the feature acquisition component is turned on. As shown in FIG. 18, the oral care position identification device includes a first acquisition module 1810 and a first determination module 1820.

The first acquisition module 1810 is configured to acquire the human body feature information during an oral care process through the feature acquisition component. The first determination module 1820 is configured to determine a current oral care position corresponding to the care element based on the human body feature information.

In one optional embodiment, the feature acquisition component includes a camera or a photoelectric sensor.

In one optional embodiment, the oral care position identification device 1800 further includes a second acquisition module and a second determination module. The second acquisition module is configured for the feature acquisition component to acquire care element feature information during the oral care process. The second determination module is configured to determine the current oral care position corresponding to the care element based on the human body feature information and the care element feature information.

In one optional embodiment, the second determination module is configured to determine a first subarea based on the human body feature information, determine a second subarea based on the care element feature information, and determine the current oral care position corresponding to the care element based on the first subarea and the second subarea. Alternatively, the second determination module is configured to determine the current oral care position corresponding to the care element based on the care element feature information and relative positioning information between the care element information and the human body feature information.

In one optional embodiment, the second determination module includes a first determination unit and a second determination unit. The first determination unit is configured to determine that the current oral care position is in an upper tooth area or a lower tooth area through the position of the care element in the care element feature information relative to the upper lip in the human body feature information. The second determination unit is configured to determine that the current oral care position is in the upper tooth area or the lower tooth area through the position of the care element in the care element feature information relative to the lower lip in the human body feature information.

In one optional embodiment, the human body feature information includes mouth feature information; and/or, the human body feature information includes chin feature information or nose feature information.

In one optional embodiment, the care element includes a connecting end and a free end opposite to the connecting end, and the connecting end of the care element is connected to the handle.

When the feature acquisition component is turned on, the feature acquisition component is able to acquire at least an image of the care element from the connecting end of the care element to a first predetermined range toward the free end of the care element, so that the feature acquisition component is able to acquire external feature information of the mouth. Optionally, when the feature acquisition component is turned on, the feature acquisition component is able to at least acquire an image of the care element from the free end of the care element to a second predetermined range toward the connecting end of the care element, so that the feature acquisition component is always able to acquire the care element feature information.

In one optional embodiment, a position of the care element of a predetermined distance from the connecting end to the free end of the care element is located at a center of the image acquired by the first positioning component, and/or the handle defines an axis, the first positioning component defines an optical axis, the axis intersects the optical axis, and at least part of the care element is within the energy field of the feature acquisition component.

In one optional embodiment, the care element extends along a first axis. The first acquisition module 1810 is specifically configured to acquire the human body feature information during the oral care process based on the feature acquisition component disposed on the handle and having the optical axis located along a second axis. An included angle between the first axis and the second axis is less than a half of the energy field of the first positioning component.

In one optional embodiment, the included angle between the first axis and the second axis is less than 45°.

In one optional embodiment, a wide angle of the feature acquisition component is 180°, and an inner deviation angle between the optical axis of the feature acquisition component and a horizontal plane is in a range of 0°-45°.

In one optional embodiment, the optical axis of the feature acquisition component is deflected inward relative to the care element. The handle includes a transition section and a holding section. A cross-sectional area of a connecting end of the handle connected to the care element is less than a cross-sectional area of the holding section. The transition section connects the holding section to the connecting end of the handle, and the feature acquisition component is disposed on the transition section. Optionally, in a circumferential dimension of the handle, when one side where a button is located or a care-oriented surface of the care element is defined as a front side of the handle, the first positioning component is disposed on a rear side of the handle, so that the human body features during the oral care process and the care elements are located on the same side of the feature acquisition component.

In one optional embodiment, the first determination module 1820 is specifically configured to input the human body feature information into an oral care position identification model, and output the current oral care position corresponding to the care element. The oral care position identification model is trained based on a large amount of human body feature information of known oral care positions.

In one optional embodiment, the human body feature information includes first human body feature information acquired when performing the oral care by a left hand and second human body feature information acquired when performing the oral care by a right hand.

In one optional embodiment, the oral care device further includes a multi-axis sensor. The multi-axis sensor is configured to acquire posture information of the oral care device. The first determination module 1820 is specifically configured to determine the current oral care position corresponding to the care element based on the human body feature information and the posture information.

In one optional embodiment, the first determination module 1820 is specifically configured to determine a third subarea based on the human body feature information, determine a fourth subarea based on the posture information, and determine the current oral care position corresponding to the care element based on the third subarea and the fourth subarea.

A division of the modules in the oral care area identification device is only for illustration. In other embodiments, the oral care area identification device may be divided into different modules as needed to complete all or part of the functions of the oral care area identification device. Each of the modules of the oral care area identification device of the embodiments of the present disclosure may be a computer program. The computer program may be run on a terminal device, a server, or the oral care device. Program modules constituted by the computer program may be stored in a memory of the terminal device, the server, or the oral care device. When the computer program is executed by the processor, all or part of the steps of the oral care identification method described in the embodiments of the present disclosure are implemented.

FIG. 19 is a schematic diagram of the oral care area identification device according to another embodiment of the present disclosure. The oral care area identification device is applied to an oral care device, and the oral care device includes a first positioning component and a second positioning component. As shown in FIG. 19, the oral care area identification device 1900 includes a first acquisition module 1910, a first judgement module 1920, a second acquisition module 1930, and a first determination module 1940.

The first acquisition module 1910 is configured to acquire current positioning information through the first positioning component. The first judgement module 1920 is configured to determine whether the first positioning component has acquired target human body feature information based on the current positioning information. The second acquisition module 1930 is configured to acquire current motion information corresponding to the oral care device based on the second positioning component when the first positioning component does not acquire the target human body feature information. The first determination module 1940 is configured to determine the current oral care area corresponding to the oral care device based on the current motion information.

In one optional embodiment, the first determination module 1940 is specifically configured to determine the current oral care area corresponding to the oral care device based on an initial oral care position acquired by the first positioning component and the current motion information.

In one optional embodiment, the oral care area identification device 1900 further includes a second determination module and a third determination module. The second determination module is configured to determine the current oral care area corresponding to the oral care device based on the current positioning information when the first positioning component acquires the target human body feature information. The third determination module is configured to determine the current oral care area corresponding to the oral care device based on the current positioning information and the current motion information.

In one optional embodiment, the oral care area identification device 1900 further includes a fourth determination module. When the first positioning component does not acquire the target human body feature information, the fourth determination module is configured to determine the initial oral care position corresponding to the oral care device based on historical positioning information acquired by the first positioning component. The historical positioning information is positioning information acquired before the first positioning component does not acquire the target human body feature information.

In one optional embodiment, the oral care area identification device 1900 further includes a second judgement module. When the first positioning component does not acquire the target human body feature information, the second judgement module is configured to determine whether there is the initial oral care position corresponding to the oral care device. When there is the initial oral care position, the second acquisition module 1930 is specifically configured to acquire the current motion information corresponding to the oral care device through the second positioning component.

In one optional embodiment, the oral care area identification device 1900 further includes a first prompt module. When there is no initial oral care position, the first prompt module is configured to issue a reminder message or stop the oral care area identification device.

In one optional embodiment, the oral care area identification device 1900 further includes a fifth determination module. When there is no initial oral care position, the fifth determination module is configured to determine the initial oral care position based on historical oral care data of the oral care device.

In one optional embodiment, the current initial oral care position is an oral care position with the highest frequency of being marked as the initial oral care position in the historical oral care data when the oral care device starts the oral care, or an oral care position corresponding to a last or previous oral care work of the oral care device in the historical oral care data.

In one optional embodiment, the current initial oral care position is an oral care guiding position displayed in a display area of a terminal connected to the oral care device or a display area of the oral care device.

In one optional embodiment, when the first positioning component is blocked, the first positioning component is unable to acquire the target human body feature information.

In one optional embodiment, the oral care area identification device 1900 further includes a sixth determination module. When a proportion of predetermined information in the current positioning information acquired by the first positioning component is greater than a predetermined proportion threshold and a change amplitude within a first predetermined time is less than a first predetermined value, the sixth determination module determines that the first positioning component is blocked.

In one optional embodiment, the oral care area identification device 1900 further includes a third acquisition module and a fourth acquisition module.

When the first positioning component is not blocked, the third acquisition module is configured to enable the first positioning component to acquire the current positioning information with a first power consumption. When the first positioning component is blocked, the fourth acquisition module is configured to enable the first positioning component to acquire the current positioning information with a second power consumption. The second power consumption is less than the first power consumption.

In one optional embodiment, the oral care area identification device 1900 further includes a second prompt module. When the first positioning component does not acquire the target human body feature information, the second prompt module is configured to issue a prompt message to remind the blockage of the first positioning component.

In one optional embodiment, the oral care area identification device 1900 further includes a fifth acquisition module. After the power of the oral care device is turned on, the fifth acquisition module is configured to enable the second positioning component to acquire the current motion information corresponding to the oral care device.

The oral care area identification device 1900 further includes a seventh determination module. When the first positioning component acquires the target human body feature information, the seventh determination module is configured to determine the current oral care area corresponding to the oral care device based on the current positioning information and the current motion information.

In one optional embodiment, when the first positioning component does not acquire the target human body feature information, the second acquisition module 1930 is specifically configured to acquire the current motion information corresponding to the oral care device through controlling the second positioning component to work at a third power consumption.

The oral care area identification device 1900 further includes a sixth acquisition module and an eighth determination module.

When the first positioning component acquires the target human body feature information, the sixth acquisition module is configured to control the second positioning component working at the fourth power consumption to acquire the current motion information corresponding to the oral care device. The eighth determination module is configured to determine the current oral care area corresponding to the oral care device based on the current positioning information and the current motion information. The third power consumption is greater than the fourth power consumption.

In one optional embodiment, after the oral care device starts the oral care work, the first acquisition module 1920 is specifically configured to acquire the current positioning information through the first positioning component.

The oral care area identification device 1900 further includes a first stop module. When the first positioning component does not acquire the target human body feature information and the oral care device stops vibrating, the first stop module is configured to control the oral care device to stop the oral care area identification.

In one optional embodiment, the current motion information includes a displacement distance. The oral care area identification device 1900 further includes a second stop module.

When the displacement distance is greater than a second predetermined value or the oral care device stops vibrating, the second stop module is configured to control the second positioning component to stop acquiring the current motion information corresponding to the oral care device.

In one optional embodiment, the first positioning component includes at least one of a camera and a structured light sensor. The second positioning component includes at least one of a multi-axis sensor, a pressure sensor, and a micro radar. The current motion information includes at least one of the posture information and the displacement information. The displacement information includes a moving direction, a flip angle, and a displacement distance of the oral care device.

A division of the modules in the oral care area identification device is only for illustration. In other embodiments, the oral care area identification device may be divided into different modules as needed to complete all or part of the functions of the oral care area identification device. Each of the modules of the oral care area identification device of the embodiments of the present disclosure may be a computer program. The computer program may be run on a terminal device, a server, or the oral care device. Program modules constituted by the computer program may be stored in a memory of the terminal device, the server, or the oral care device. When the computer program is executed by the processor, all or part of the steps of the oral care identification method described in the embodiments of the present disclosure are implemented.

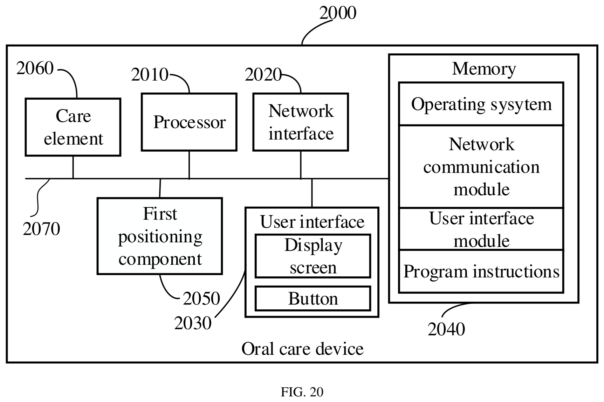

FIG. 20 is a block diagram of the oral care device according to one embodiment of the present disclosure. As shown in FIG. 20, the oral care device 2000 includes at least one processor 2010, a care element 2060 a user interface 2030, a memory 2040, a feature acquisition component 2050, and a communication bus 2070.

The communication bus 2070 is configured to realize connection and communication between the various components of the oral care device 2000.

Optionally, the at least one network interface 950 includes a BLUETOOTH module, a near field communication (NFC) module, a wireless fidelity (Wi-Fi) module, etc. The user interface 2030 includes a display screen and a button. Optionally, the user interface 2030 may further include a standard wired interface and a wireless interface.

The feature acquisition component 2050 is configured to acquire images or light energy information, and feature acquisition component 2050 is always able to acquire care element feature information of at least part of the care element 2060 when turned on.

The at least one processor 2010 includes one or more processing cores. The at least one processor 2010 uses various interfaces and lines to connect various parts of the oral care device 2000. The processor 2010 executes various functions and processes data of the oral care device 2000 by running or executing instructions, programs, code sets or instruction sets stored in the memory 2040, and calling data stored in the memory 2040. Optionally, the at least one processor 2010 is implemented by at least one hardware of a digital signal processing (DSP), a field programmable gate array (FPGA), and a programmable logic array (PLA). The at least one processor 2010 may integrate one or a combination of a central processing unit (CPU), a graphics processing unit (GPU), and a modem. The CPU mainly processes an operating system and an application for the oral care device 2000. The GPU is responsible for rendering and drawing content to be displayed on the display screen. The modem is configured to process wireless communications. It is understood that the modem may not be integrated into the at least one processor 2010, but implemented by a chip.

The memory 2040 may be a random access memory (RAM) or a read-only memory (ROM). Optionally, the memory 2040 may be a non-transitory computer-readable medium. The memory 2040 is configured to store the instructions, the programs, the codes, the code sets or the instruction sets. The memory 2040 includes a program storage area and a data storage area. The program storage area stores the instructions for implementing the operating system, the instructions for at least one function (such as a receiving function, a control function, a determination function, etc.), the instructions for implementing the controls method of the present disclosure, etc. The data storage area stores data involved in the control method of the present disclosure, etc. Optionally, the memory 2040 may be at least one storage device located away from the at least one processor 2010. As shown in FIG. 20, the memory 2040 served as a computer storage medium may include the operating system, a network communication module, a user interface module, and program instructions.

In some optional embodiments, the at least one processor 2010 may be configured to call program instructions stored in the memory 2040 and specifically execute the oral care area identification method described in the aforementioned embodiment, which is not described in detail herein.

In some optional embodiments, the at least one processor 2010 is further configured to execute steps of acquiring human body feature information of a user during an oral care process through a first positioning component of the oral care device and determining a current oral care position corresponding to a care element of the oral care device based on the human body feature information.

In some optional embodiments, when the at least one processor 2010 executes the steps of acquiring the human body feature information of the user during the oral care process through the first positioning component of the oral care device and determining the current oral care position corresponding to the care element of the oral care device based on the human body feature information, the at least one process is specifically configured to execute steps of determining the first subarea based on the human body feature information, determining a second subarea based on the care element feature information, and determining the current oral care position corresponding to the care element based on the first subarea and the second subarea; or determining the current oral care position corresponding to the care element based on the care element feature information and relative information of the human body feature information.

In some optional embodiments, when the at least one processor 2010 executes the steps of determining the current oral care position corresponding to the care element based on the care element feature information and the relative information of the human body feature information, the at least one processor 2010 specifically executes steps of determining whether the current oral care position is in an upper tooth area or a lower tooth area through the position of the care element relative to an upper lip; or determining whether the current oral care position is in the upper tooth area or the lower tooth area through the position of the care element relative to a lower lip.

In some optional embodiments, the first positioning component includes a camera or a photoelectric, and/or the human body feature information includes mouth feature information; and/or the human body feature information includes chin feature information or nose feature information.

In some optional embodiments, the oral care device includes a handle, the care element includes a connecting end and a free end opposite to the free end, and the connecting end of the care element is connected to the handle. When the first positioning component is turned on, the first positioning component is able to acquire at least an image of the care element from the connecting end of the care element to a first predetermined range toward the free end of the care element, so that the first positioning component is able to acquire external feature information of the mouth. In some embodiments, when the first positioning component is turned on, the first positioning component is able to at least acquire an image of the care element from the free end of the care element to a second predetermined range toward the connecting end of the care element, so that the first positioning component is always able to acquire the care element feature information.

In some optional embodiments, a position of the care element of a predetermined distance from the connecting end to the free end of the care element is located at a center of the image acquired by the first positioning component. In some optional embodiments, the handle defines an axis, the first positioning component defines an optical axis, the axis intersects the optical axis, and at least part of the care element is within the FOV of the first positioning component; and/or

In some optional embodiments, the oral care device further includes a handle, and the care element extends along a first axis. When the at least one processor 2010 executes the step of acquiring the human body feature information of the user during the oral care process through the first positioning component of the oral care device, the at least one processor specifically executes a step of acquiring the human body feature information during the oral care process based on the first positioning component disposed on the handle and having an optical axis located on a second axis. An included angle between the second axis and the first axis is less than half of a wide angle of the first positioning component.

In some optional embodiments, the included angle between the first axis and the second axis is less than 45°; and/or the optical axis of the first positioning component is deflected inward relative to the care element; and/or the wide angle of the first positioning component is 180°, and an inner deviation angle between the optical axis of the first positioning component and a horizontal plane is in a range of 0°-45°.

In some optional embodiments, the oral care device includes a handle. The handle includes a transition section and a holding section. A cross-sectional area of a connecting end of the handle connected to the care element is less than a cross-sectional area of the holding section, the transition section connects the holding section to the connecting end of the handle, and the first positioning component is disposed on the transition section; and/or in a circumferential dimension of the handle, when one side where a button is located or a care-oriented surface of the care element is defined as a front side of the handle, the first positioning component is disposed on a rear side of the handle.

In some optional embodiments, when the at least one processor executes the step of determining the current oral care position corresponding to the care element of the oral care device based on the human body feature information, the at least one processor specifically executes a step of inputting the human body feature information into an oral care position identification model, and outputting the current oral care position corresponding to the care element. The oral care position identification model is trained based on a large amount of human body feature information of known oral care positions.

In some optional embodiments, the human body feature information includes first human body feature information acquired when performing the oral care by a left hand and second human body feature information acquired when performing the oral care by a right hand.

In some optional embodiments, the oral care device further includes a multi-axis sensor, and the multi-axis sensor is configured to acquire posture information of the oral care device. When the at least one processor 2010 executes the step of determining the current oral care position corresponding to the care element of the oral care device based on the human body feature information, the at least one processor executes a step of determining the current oral care position corresponding to the care element based on the human body feature information and the posture information.

In some optional embodiments, when the at least one processor 2010 executes the step of determining the current oral care position corresponding to the care element based on the human body feature information and the posture information, the at least one processor executes steps of determining a third subarea based on the human body feature information; determining a fourth subarea based on the posture information; and determining the current oral care position corresponding to the care element based on the third subarea and the fourth subarea.

In some optional embodiments, the oral care device 2000 shown in FIG. 20 is the oral care device. The oral care device further includes a second positioning component. The at least one processor 2010 is configured to call the program instructions stored in the memory 2040 and specifically executes steps of acquiring current positioning information based on the first positioning component; determining whether the first positioning component acquires target human body feature information based on the current positioning information; and when the first positioning component does notacquireacquire the target human body feature information, acquiring current motion information corresponding to the oral care device based on the second positioning component, and determining the current oral care area corresponding to the oral care device based on the current motion information.

In some optional embodiments, when the at least one processor performs the step of determining the current oral care area corresponding to the oral care device based on the current motion information, the at least one processor specifically executes a step of determining the current oral care area corresponding to the oral care device based on an initial oral care position acquired by the first positioning component and the current motion information.

In some optional embodiments, after the at least one processor 2010 executes the step of determining whether the first positioning component acquires the target human body feature information based on the current positioning information, the at least one processor 2010 further executes steps: when the first positioning component acquires the target human body feature information, determining the current oral care area corresponding to the oral care device based on the current positioning information; or determining the current oral care area corresponding to the oral care device based on the current positioning information and the current motion information; or when the first positioning component does not acquire the target human body feature information, issuing a prompt message to remind the blockage of the first positioning component.

In some optional embodiments, the at least one processor 2010 further executes a step of determining an initial oral care position corresponding to the oral care device based on historical positioning information acquired by the first positioning component when the first positioning component does not acquire the human body feature information. The historical positioning information is positioning information acquired by the first positioning component before acquiring the human body feature information.

In some optional embodiments, the at least one processor 2010 further executes a step: when the first positioning component does not acquire the human body feature information, determining whether there is an initial oral care position corresponding to the oral care device.

When the at least one processor executes the step of acquiring the current motion information corresponding to the oral care device through the second positioning component, the at least one processor specifically executes a step: when there is the initial oral care position, acquiring the current motion information corresponding to the oral care device through the second positioning component.

In some optional embodiments, after the at least one processor 2010 further executes the step of determining whether there is the initial oral care position corresponding to the oral care device when the first positioning component does not acquire the target human body feature information, the at least one processor further executes a step of issuing a reminder message or stopping the oral care device when there is no initial oral care position.

In some optional embodiments, after executing the step of determining whether there is the initial oral care position corresponding to the oral care device when the first positioning component does not acquire the target human body feature information by the at least one processor and before executing the step of acquiring the current motion information corresponding to the oral care device through the second positioning component, the at least one processor 2010 further executes a step: when there is no initial oral care position, determining a current initial oral care position based on historical oral care data of the oral care device.

In some optional embodiments, the current initial oral care position is an oral care position with the highest frequency of being marked as the initial oral care position in the historical oral care data when the oral care device starts the oral care, or the current initial oral care position is an oral care position corresponding to a last or previous oral care work of the oral care device in the historical oral care data.

In some optional embodiments, the current initial oral care position is an oral care guiding position displayed in a display area of a terminal connected to the oral care device or a display area of the oral care device.

In some optional embodiments, when the first positioning component is blocked, the first positioning component is unable to acquire the target human body feature information.

In some optional embodiments, the at least one processor further executes a step of determining that the first positioning component is blocked when a proportion of predetermined information in the current positioning information acquired by the first positioning component is greater than a predetermined proportion threshold and a change amplitude within a first predetermined time is less than a first predetermined value.

In some optional embodiments, the at least one processor further executes steps of controlling the first positioning component to acquire the current positioning information with a first power consumption when the first positioning component is not blocked; and controlling the first positioning component to acquire the current positioning information with a second power consumption when the first positioning component is blocked. The second power consumption is less than the first power consumption.

In some optional embodiments, the at least one processor further executes steps of after a power source of the oral care device is turned on, acquiring the current motion information corresponding to the oral care device by the second positioning component.

After the at least one processor executes the step of determining whether the first positioning component acquires the target human body feature information based on the current positioning information, the at least one processor further executes a step: when the first positioning component acquires the target human body feature information, determining the current oral care area corresponding to the oral care device based on the current positioning information and the current motion information.

In some optional embodiments, when the at least one processor executes the step of when the first positioning component does not acquire the target human body feature information, acquiring the current motion information corresponding to the oral care device based on the second positioning component, the at least one processor further executes a step of when the first positioning component does not acquire] the target human body feature information, acquiring the current motion information corresponding to the oral care device based on the second positioning component operating at a third power consumption.

After the at least one processor executes the step of determining whether the first positioning component acquires the target human body feature information based on the current positioning information, the at least one processor 2010 further executes steps of when the first positioning component acquires the target human body feature information, acquiring current motion information corresponding to the oral care device based on the second positioning component operating at a fourth power consumption; and determining the current oral care area corresponding to the oral care device based on the current positioning information and the current motion information. The third power consumption is greater than the fourth power consumption.

In some optional embodiments, when the at least one processor executes the step of acquiring the current positioning information based on the first positioning component, the at least one processor specifically executes a step of after the oral care device starts the oral care, acquiring the current positioning information based on the first positioning component.

After the at least one processor executes the step of determining whether the first positioning component acquires the target human body feature information based on the current positioning information, the at least one processor 2010 further executes a step: when the first positioning component does not acquire the target human body feature information, and the oral care device stops vibrating, stopping the oral care area identification operation by the oral care device.

In some optional embodiments, the current motion information includes a displacement distance. After the at least one processor executes the step of acquiring the current motion information corresponding to the oral care device based on the second positioning component, the at least one processor 2010 further executes a step of when the displacement distance is greater than a second predetermined value or the oral care device stops vibrating, the second positioning component stops acquiring the current motion information corresponding to the oral care device.

In some optional embodiments, the first positioning component includes at least one of a camera and a structured light sensor. The second positioning component includes at least one of a multi-axis sensor, a pressure sensor, and a micro radar. The current motion information includes at least one of posture information and displacement information. The displacement information includes a moving direction, a flip angle, and a displacement distance.

The embodiment of the present disclosure further provides a computer storage medium, and the instructions are stored in the computer storage medium. When the instructions are executed on a computer or the at least one processor, the computer or the at least one processor executes one or more steps of the oral care area identification method in any of the embodiments. When component modules of the oral care area identification device are implemented in a form of software functional units and are sold or used as an independent product, the component modules are stored in the computer storage medium.

In the above embodiments, the present disclosure may be implemented in whole or in part by software, hardware, firmware or any combination thereof. When implemented by software, the software is implemented in whole or in part as a form of a computer program product. The computer program product includes one or more computer instructions. When the one or more computer program instructions are loaded and executed on the computer, the process or function described in the embodiments of the present disclosure is generated in whole or in part. The computer may be a general-purpose computer, a special-purpose computer, a computer network, or other programmable device. The one or more computer instructions may be stored in the computer-readable storage medium or transmitted through the computer-readable storage medium. The one or more computer instructions may be transmitted from a website site, a computer, a server, or a data center to another website site, another computer, another server or another data center by wired (e.g., coaxial cable, optical fiber, digital subscriber line) or wireless (e.g., infrared, wireless, microwave, etc.) mode. The computer-readable storage medium may be any available medium that is able to be accessed by the computer or a data storage device such as the server or the data center that includes one or more available media integrated. The one or more available media may be a magnetic medium (e.g., a floppy disk, a hard disk, a magnetic tape), an optical medium (e.g.,, a digital versatile disc (DVD)), or a semiconductor medium (e.g., a solid state disk (SSD)).

Those skilled in the art can understand that all or part of the processes in the above-mentioned embodiments can be implemented by instructing the relevant hardware through the computer programs, and the computer programs may be stored in the computer-readable storage medium. When the computer programs are executed, the processes of the embodiments of the present disclosure are executed. The storage medium includes the ROM, the RAM, a magnetic disk, an optical disk, or other media that are able to store the program codes. In the absence of conflict, the technical features in the embodiments and implementation schemes of the present disclosure can be combined arbitrarily

The embodiments described above are merely optional embodiments of the present disclosure and are not intended to limit the scope of the present disclosure. Without departing from the design spirit of the present disclosure, various modifications and improvements made to the technical solutions of the present disclosure by those skilled in the art should fall within the protection scope of the claims of the present disclosure.

## Claims

1. An oral care position identification method applied to an oral care device, comprising steps:
acquiring human body feature information of a user during an oral care process through a first positioning component of the oral care device; and
determining a current oral care position corresponding to a care element of the oral care device based on the human body feature information;
wherein the oral care device comprises a handle, the care element, and the first positioning component disposed on the handle, the first positioning component is configured to acquire images and energy information, when the first positioning component is turned on, the first positioning component is always able to acquire feature information of at least part of the care element.

2. The oral care position identification method according to claim 1, wherein the oral care position identification method further comprises steps:
acquiring care element feature information during the oral care process based on the first positioning component;
and
determining the current oral care position corresponding to the care element based on the human body feature information and the care element feature information.

3. The oral care position identification method according to claim 2, wherein the step of determining the current oral care position corresponding to the care element based on the human body feature information and the care element feature information comprises steps:
determining a first subarea based on the human body feature information, determining a second subarea based on the care element feature information, and determining the current oral care position corresponding to the care element based on the first subarea and the second subarea; or
determining the current oral care position corresponding to the care element based on the care element feature information and relative information of the human body feature information.

4. The oral care position identification method according to claim 3, wherein the step of determining the current oral care position corresponding to the care element based on the care element feature information and the relative information of the human body feature information comprises steps:
determining whether the current oral care position is in an upper tooth area or a lower tooth area through a position of the care element relative to an upper lip; or
determining whether the current oral care position is in the upper tooth area or the lower tooth area through the position of the care element relative to a lower lip.

5. The oral care position identification method according to claim 1, wherein the first positioning component comprises a camera or a photoelectric, and/or the human body feature information comprises mouth feature information.

6. The oral care position identification method according to claim 5, wherein the human body feature information comprises chin feature information or nose feature information.

7. The oral care position identification method according to claim 1, wherein the care element comprises a connecting end and a free end opposite to the free end, and the connecting end of the care element is connected to the handle;
wherein when the first positioning component is turned on, the first positioning component is able to acquire at least an image of the care element from the connecting end of the care element to a first predetermined range toward the free end of the care element, so that the first positioning component is able to acquire external feature information of the mouth; and/or
when the first positioning component is turned on, the first positioning component is able to at least acquire an image of the care element from the free end of the care element to a second predetermined range toward the connecting end of the care element, so that the first positioning component is always able to acquire the care element feature information.

8. The oral care position identification method according to claim 7, wherein a position of the care element of a predetermined distance from the connecting end to the free end of the care element is located at a center of the image acquired by the first positioning component

9. The oral care position identification method according to claim 1, wherein the handle defines an axis, the first positioning component defines an optical axis, the axis intersects the optical axis, and at least part of the care element is within the FOV of the first positioning component; and/or
the handle comprises a transition section and a holding section, a cross-sectional area of a connecting end of the handle connected to the care element is less than a cross-sectional area of the holding section, the transition section connects the holding section to the connecting end of the handle, and the first positioning component is disposed on the transition section; and/or
in a circumferential dimension of the handle, when one side where a button is located or a care-oriented surface of the care element is defined as a front side of the handle, the first positioning component is disposed on a rear side of the handle.

10. The oral care position identification method according to claim 1, wherein the care element extends along a first axis; wherein the step of acquiring the human body feature information of the user during the oral care process through the first positioning component of the oral care device comprises:
acquiring the human body feature information during the oral care process based on the first positioning component disposed on the handle and having an optical axis located on a second axis, wherein an included angle between the second axis and the first axis is less than half of a wide angle of the first positioning component.

11. The oral care position identification method according to claim 10, wherein the included angle between the first axis and the second axis is less than 45°; and/or
the optical axis of the first positioning component is deflected inward relative to the care element; and/or
the wide angle of the first positioning component is 180°, and an inner deviation angle between the optical axis of the first positioning component and a horizontal plane is in a range of 0°-45°.

12. The oral care position identification method according to claim 1, wherein the step of determining the current oral care position corresponding to the care element of the oral care device based on the human body feature information comprises
inputting the human body feature information into an oral care position identification model, and outputting the current oral care position corresponding to the care element,
wherein the oral care position identification model is trained based on a large amount of human body feature information of known oral care positions.

13. The oral care position identification method according to claim 12, wherein the human body feature information comprises first human body feature information acquired when performing the oral care by a left hand and second human body feature information acquired when performing the oral care by a right hand.

14. The oral care position identification method according to claim 1, wherein the oral care device further comprises a multi-axis sensor, the multi-axis sensor is configured to acquire posture information of the oral care device;
wherein the step of determining the current oral care position corresponding to the care element of the oral care device based on the human body feature information comprises a step:
determining the current oral care position corresponding to the care element based on the human body feature information and the posture information.

15. The oral care position identification method according to claim 14, wherein the step of determining the current oral care position corresponding to the care element based on the human body feature information and the posture information comprises steps:
determine a third subarea based on the human body feature information;
determining a fourth subarea based on the posture information; and
determine the current oral care position corresponding to the care element based on the third subarea and the fourth subarea.

16. The oral care position identification method according to claim 1, wherein the oral care device further comprises a second positioning component; wherein the oral care position identification method further comprises steps:
acquiring current positioning information based on the first positioning component; determining whether the first positioning component acquires target human body feature information based on the current positioning information; and
when the first positioning component does not acquire the target human body feature information, acquiring current motion information corresponding to the oral care device based on the second positioning component, and determining the current oral care area corresponding to the oral care device based on the current motion information.

17. The oral care position identification method according to claim 16, wherein the step of determining the current oral care area corresponding to the oral care device based on the current motion information comprises determining the current oral care area corresponding to the oral care device based on an initial oral care position acquired by the first positioning component and the current motion information.

18. The oral care position identification method according to claim 16, wherein after the step of determining whether the first positioning component acquires the target human body feature information based on the current positioning information, the oral care position identification method further comprises steps:
when the first positioning component acquires the target human body feature information, determining the current oral care area corresponding to the oral care device based on the current positioning information; or
determining the current oral care area corresponding to the oral care device based on the current positioning information and the current motion information; or
when the first positioning component does not acquire the target human body feature information, issuing a prompt message to remind the blockage of the first positioning component

19. The oral care position identification method according to claim 16, wherein the oral care position identification method further comprises a step:
determining an initial oral care position corresponding to the oral care device based on historical positioning information acquired by the first positioning component when the first positioning component does not acquire the human body feature information; wherein the historical positioning information is positioning information acquired by the first positioning component before acquiring the human body feature information.

20. The oral care position identification method according to claim 16, wherein the oral care position identification method further comprises a step:
when the first positioning component does not acquire the human body feature information, determining whether there is an initial oral care position corresponding to the oral care device;
wherein the step of acquiring the current motion information corresponding to the oral care device through the second positioning component comprises a step:
when there is the initial oral care position, acquiring the current motion information corresponding to the oral care device through the second positioning component.

21. The oral care position identification method according to claim 20, wherein after the step of determining whether there is the initial oral care position corresponding to the oral care device when the first positioning component does not acquire the target human body feature information, the oral care area identification method further comprises a step
issuing a reminder message or stopping the oral care device when there is no initial oral care position.

22. The oral care position identification method according to claim 20, wherein after the step of determining whether there is the initial oral care position corresponding to the oral care device when the first positioning component does not acquire the target human body feature information and before the step of acquiring the current motion information corresponding to the oral care device through the second positioning component, the oral care area identification method further comprises a step:
when there is no initial oral care position, determining a current initial oral care position based on historical oral care data of the oral care device.

23. The oral care position identification method according to claim 22, wherein the current initial oral care position is an oral care position with the highest frequency of being marked as the initial oral care position in the historical oral care data when the oral care device starts the oral care, or
the current initial oral care position is an oral care position corresponding to a last or previous oral care work of the oral care device in the historical oral care data.

24. The oral care position identification method according to claim 20, wherein the current initial oral care position is an oral care guiding position displayed in a display area of a terminal connected to the oral care device or a display area of the oral care device.

25. The oral care position identification method according to claim 16, wherein when the first positioning component is blocked, the first positioning component is unable to acquire the target human body feature information.

26. The oral care position identification method according to claim 25, wherein the oral care position identification method further comprises a step:
determining that the first positioning component is blocked when a proportion of predetermined information in the current positioning information acquired by the first positioning component is greater than a predetermined proportion threshold and a change amplitude within a first predetermined time is less than a first predetermined value.

27. The oral care position identification method according to claim 16, wherein the oral care position identification method further comprises steps:
controlling the first positioning component to acquire the current positioning information with a first power consumption when the first positioning component is not blocked;
controlling the first positioning component to acquire the current positioning information with a second power consumption when the first positioning component is blocked;
wherein the second power consumption is less than the first power consumption.

28. The oral care position identification method according to claim 16, wherein the oral care position identification method further comprises steps:
after a power source of the oral care device is turned on, acquiring the current motion information corresponding to the oral care device by the second positioning component.
wherein after the step of determining whether the first positioning component acquires the target human body feature information based on the current positioning information, the oral care position identification method further comprises a step:
when the first positioning component acquires the target human body feature information, determining the current oral care area corresponding to the oral care device based on the current positioning information and the current motion information.

29. The oral care position identification method according to claim 16, wherein the step of when the first positioning component does not acquire the target human body feature information, acquiring the current motion information corresponding to the oral care device based on the second positioning component comprises a step:
when the first positioning component does not acquire the target human body feature information, acquiring the current motion information corresponding to the oral care device based on the second positioning component operating at a third power consumption; and
after the step of determining whether the first positioning component acquires the target human body feature information based on the current positioning information, the oral care position identification method further comprises a step:
when the first positioning component acquires the target human body feature information, acquiring current motion information corresponding to the oral care device based on the second positioning component operating at a fourth power consumption; and
determining the current oral care area corresponding to the oral care device based on the current positioning information and the current motion information;
wherein the third power consumption is greater than the fourth power consumption.

30. The oral care position identification method according to claim 16, wherein the step of acquiring the current positioning information based on the first positioning component comprises a step:
after the oral care device starts the oral care, acquiring the current positioning information based on the first positioning component;
wherein after the step of determining whether the first positioning component acquires the target human body feature information based on the current positioning information, the oral care position identification method further comprises a step:
when the first positioning component does not acquire the target human body feature information, and the oral care device stops vibrating, stopping the oral care area identification operation by the oral care device.

31. The oral care position identification method according to claim 16, wherein the current motion information comprises a displacement distance;
wherein after the step of acquiring the current motion information corresponding to the oral care device based on the second positioning component, the oral care position identification method further comprises a step:
when the displacement distance is greater than a second predetermined value or the oral care device stops vibrating, the second positioning component stops acquiring the current motion information corresponding to the oral care device.

32. The oral care position identification method according to any one of claims 16-31, wherein the first positioning component comprises at least one of a camera and a structured light sensor; the second positioning component comprises at least one of a multi-axis sensor, a pressure sensor, and a micro radar; and the current motion information comprises at least one of posture information and displacement information; the displacement information comprises a moving direction, a flip angle, and a displacement distance.

33. An oral care position identification device, wherein the oral care position identification device is an oral care device comprising:
a handle;
a care member;
a first positioning component
a first acquiring module, and
a first determining module,
wherein the first positioning component is disposed on the handle and is configured to acquire image or light energy information, the first positioning component is able to acquire at least part of care element feature information when being turned on, the first acquiring module is configured to acquire human body feature information during an oral care process based on the first positioning component; and the first determining module is configured to determine a current oral care position corresponding to the care member based on the human body feature information.

34. An oral care device, comprising:
a memory;
a processor;
a handle;
a care element; and
a first positioning component;
wherein the first positioning component is disposed on the handle and is configured to acquire images and energy information, when the first positioning component is turned on, the first positioning component is always able to acquire feature information of at least part of the care element;
wherein the memory is connected to the processor and is configured to store executable program codes;
wherein the processor is configured to run a program corresponding to the executable program codes by reading the executable program codes stored in the memory, and the processor is configured to execute steps:
acquiring human body feature information of a user during an oral care process through the first positioning component of the oral care device; and
determining a current oral care position corresponding to the care element based on the human body feature information.

35. A computer storage medium, comprising:
instructions stored therein;
wherein the instructions are loaded by the processor and executed to acquire human body feature information during an oral care process based on a first positioning component of an oral care device, and determine a current oral care position corresponding to a care element of the oral care device based on the human body feature information.
